# EUROPÄISCHE PATENTANMELDUNG

(11) **EP 4 847 019 A1**
(43) Veröffentlichungstag der Anmeldung: **02.12.2026**
(21) Anmeldenummer: 26180949.5
(22) Anmeldetag: 26.05.2026
(51) Int. Cl.: A61K 31/198, A61K 9/00, A61K 31/53, A61K 31/785, A61K 45/06, A61P 17/02

(54) **ZUSAMMENSETZUNG ZUR ANWENDUNG BEI DER BEHANDLUNG VON WUNDEN UND/ODER VORBEUGUNG VON NEKROSEN**

(30) Priorität: 28.05.2025 DE 102025120930
(71) Anmelder: B. Braun Melsungen AG, 34212 Melsungen (DE)
(72) Erfinder: Schmidt, Yuliya, 4310 Rheinfelden (CH); Henze, Heiko, 4310 Rheinfelden (CH)
(74) Vertreter: Patentanwälte Ruff, Wilhelm, Beier, Dauster & Partner mbB

(57) **Zusammenfassung**

Die Erfindung betrifft eine Zusammensetzung zur Anwendung bei der Behandlung von Wunden und/oder Vorbeugung oder Behandlung von Nekrosen, wobei die Zusammensetzung L-Arginin und/oder ein Salz von L-Arginin aufweist.

## Beschreibung

Die Erfindung betrifft eine Zusammensetzung zur Anwendung bei der Behandlung von Wunden und/oder Vorbeugung von Nekrosen.

Die Behandlung von Wunden stellt eine große Herausforderung in der täglichen Versorgungsmedizin dar. Derzeit verfügbare Präparate enthalten beispielsweise Polyhexanid, Chlorhexidin oder Octenidin und werden für die Spülung und/oder Reinigung von Wunden eingesetzt. Obgleich gute Reinigungsergebnisse erzielbar sind, besteht vor allem bei chronischen Wunden das prinzipielle Risiko, dass kein eindeutiger Heilungsverlauf erkennbar ist und schlimmstenfalls sogar Nekrosen entstehen können.

### AUFGABE UND LÖSUNG

Aufgabe der Erfindung ist es, eine Zusammensetzung bereitzustellen, die die vorgenannten Risiken bei der Behandlung von Wunden adressiert.

Diese Aufgabe wird gelöst durch eine Zusammensetzung gemäß unabhängigem Anspruch 1. Bevorzugte Ausgestaltungen der Zusammensetzung sind in den abhängigen Ansprüchen definiert. Der Wortlaut sämtlicher Ansprüche wird hiermit durch ausdrückliche Bezugnahme zum Inhalt der vorliegenden Beschreibung gemacht.

Die Erfindung betrifft eine Zusammensetzung für die Behandlung von Wunden, insbesondere chronischen Wunden, und/oder für die Vorbeugung oder Behandlung von Nekrosen. Insbesondere betrifft die Erfindung eine Zusammensetzung zur Anwendung bei der Behandlung von Wunden, insbesondere chronischen Wunden, und/oder zur Anwendung bei der Vorbeugung oder Behandlung von Nekrosen. Bei den Wunden kann es sich grundsätzlich um menschliche oder nichtmenschliche Wunden handeln. Vorzugsweise handelt es sich bei den Wunden um menschliche Wunden.

Bevorzugt handelt es sich bei der Zusammensetzung um eine pharmazeutische Zusammensetzung oder ein Medizinprodukt. Alternativ kann es sich bei der Zusammensetzung um eine kosmetische Zusammensetzung handeln.

Die Zusammensetzung zeichnet sich besonders dadurch aus, dass sie L-Arginin und/oder ein Salz, insbesondere ein bioverträgliches und/oder pharmazeutisch akzeptables Salz, von L-Arginin aufweist. Bei dem Salz von L-Arginin kann es sich beispielsweise um das Hydrochlorid von L-Arginin, d.h. um L-Arginin-Hydrochlorid, handeln.

Unter dem Ausdruck "chronische Wunde" soll im Sinne der vorliegenden Erfindung eine Wunde verstanden werden, die nach 4 Wochen bis 12 Wochen, insbesondere 6 Wochen bis 12 Wochen, vorzugsweise nach 12 Wochen, noch nicht verschlossen oder epithelisiert ist oder nach einer zwei- bis vierwöchigen, insbesondere drei- bis vierwöchigen, vorzugsweise vierwöchigen, Behandlung keine Heilungstendenz zeigt.

Unter dem Ausdruck "Nekrose" soll im Sinne der vorliegenden Erfindung allgemeinen das pathologische Absterben von Zellen, insbesondere der Tod von Zellen durch Schädigung der Zellstruktur, zum Beispiel infolge mechanischer Verletzungen, Kontakt mit Toxinen, Hypoxie, Hypothermie oder Infektionen mit Krankheitserregern, und/oder der mikroskopisch oder makroskopisch sichtbare Untergang, d.h. Zerfall, von Gewebe in einem lebenden Organismus, verstanden werden.

Unter dem Ausdruck "L-Arginin" soll im Sinne der vorliegenden Erfindung eine proteinogene α-Aminosäure mit nachfolgender Strukturformel verstanden werden:

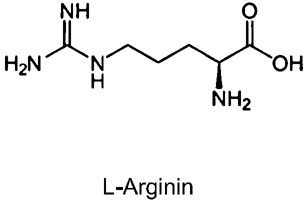

Die Erfindung beruht auf der überraschenden Erkenntnis, dass eine Behandlung von Wunden, insbesondere chronischen Wunden, und insbesondere die Vermeidung von Nekrosen durch eine Verabreichung von L-Arginin und/oder einem Salz von L-Arginin auf die Wunden erzielbar ist. Der heilungsfördernde Einfluss von L-Arginin und/oder einem Salz von L-Arginin kann anhand der nachfolgend beschriebenen Prozesse erklärt werden. In Wunden werden aufgrund eigener biochemischer Abläufe sogenannte reaktive Sauerstoff-Spezies ("reactive oxygen species", ROS) erzeugt. Hierbei handelt es sich um reaktive sauerstoffhaltige Metaboliten, die in Form von Radikalen, wie beispielsweise Hyperoxid-Anion, Hydroxyl-Radikal, Peroxyl-Radikal und/oder Alkoxy-Radikal von Lipiden, und/oder stabilen molekularen Oxidantien, wie beispielsweise Wasserstoffperoxid, Hydroperoxid, Ozon und/oder Hypochlorit-Anion, und/oder angeregten Sauerstoffmolekülen vorliegen können. Die reaktiven Sauerstoffspezies entstehen, wenn Gewebe mechanischen, chemischen oder metabolischen Stoffwechselreaktionen ausgesetzt sind. Mit L-Arginin und/oder einem Salz von L-Arginin können die reaktiven Sauerstoffspezies zu Stickstoffmonoxid (NO) und L-Citrullin reagieren. Stickstoffmonoxid führt physiologisch zu einer Blutgefäßerweiterung, indem das Stickstoffmonoxid in die Muskelschicht der Blutgefäße diffundiert. Es aktiviert dort die lösliche Guanylatcyclase und führt so zur Erschlaffung der glatten Muskulatur und zum Nachlassen des Blutgefäßtonus. Durch die Blutgefäßerweiterung kann eine lokale Durchblutung in den Wunden gefördert werden. Eine bessere Durchblutung wiederum fördert die Entstehung neuer Blutgefäße (Angiogenese) sowie stimuliert die Zellteilung. Dies begünstigt insgesamt den Wundheilungsverlauf, was insbesondere bei chronischen Wunden vorteilhaft ist. Ferner lässt sich hierdurch die Bildung von Nekrosen verhindern oder das Risiko für die Entstehung von Nekrosen wenigstens signifikant reduzieren.

Da bei der oben beschriebenen Metabolisierung von L-Arginin und/oder einem Salz von L-Arginin oxidative/reduzierende Prozesse stattfinden, kann vorteilhafterweise zusätzlich ein antimikrobieller, insbesondere antibakterieller, sowie insbesondere entzündungshemmender Effekt erzielt werden.

Weiter ist von Vorteil, dass L-Arginin und/oder ein Salz von L-Arginin sehr gute feuchtigkeitsspendende Eigenschaften besitzt, was für einen erfolgreichen Wundheilungsverlauf ebenfalls sehr förderlich ist.

In Ausgestaltung der Erfindung weisen/weist das L-Arginin und/oder Salz von L-Arginin einen Anteil von 1 Gew.-% bis 40 Gew.-%, insbesondere 2 Gew.-% bis 20 Gew.-%, vorzugsweise 5 Gew.-% bis 15 Gew.-%, auf, bezogen auf das Gesamtgewicht der Zusammensetzung. Die erfindungsgemäßen Vorteile kommen insbesondere bei den in diesem Absatz genannten Anteilen für L-Arginin und/oder das Salz von L-Arginin besonders stark zur Geltung.

In weiterer Ausgestaltung der Erfindung weist die Zusammensetzung ferner einen antimikrobiellen, insbesondere antibakteriellen, Wirkstoff auf. Der antimikrobielle Wirkstoff kann insbesondere ausgewählt sein aus der Gruppe bestehend aus Polyhexanid (Polyhexamethylenbiguanid), Chlorhexidin (1,1'-Hexamethylenbis[5-(4-chlorphenyl)biguanid]) , Octanid, Povidon-lod, Ethacridinlactat, Octenidin (N-Octyl-1-[10-(4-octyliminopyridin-1-yl)decyl]pyridin-4-imin), Polylysin, Dihydrotriazinverbindungen wie Femotaxidin (4-Octylamino-1,6-dihydro-6,6-dimethyl-2-(4'-methylbenzylamino)-1,3,5-triazin-D-gluconat) und Mischungen von wenigstens zwei der vorgenannten antimikrobiellen Wirkstoffe. Durch einen antimikrobiellen Wirkstoff können der Zusammensetzung vorteilhafterweise, insbesondere zusätzliche, antimikrobielle, insbesondere antibakterielle und/oder biofilmauflösende, Eigenschaften verliehen werden, wodurch der Wundheilungsverlauf zusätzlich gefördert werden kann. Besonders bevorzugt handelt es sich bei dem antimikrobiellen Wirkstoff um Polyhexanid bzw. Polyhexamethylenbiguanid.

Unter dem Ausdruck "Dihydrotriazinverbindungen" sollen im Sinne der vorliegenden Erfindung Verbindungen mit einem sechsgliedrigen, lediglich zwei Doppelbindungen aufweisenden Triazinring, d.h. einem sogenannten Dihydrotriazinring, verstanden werden, wobei der Triazinring bzw. Dihydrotriazinring an den Positionen 1, 3 und 5 jeweils ein Ringstickstoffatom und an den Positionen 2, 4, und 6 jeweils ein Ringkohlenstoffatom aufweist und wobei die Stellung der einen Doppelbindung zwischen den Positionen 4 und 5 des Triazinings bzw. Dihydrotriazinrings und die Stellung der anderen, d.h. zweiten oder verbleibenden, Doppelbindung entweder zwischen den Positionen 1 und 2 oder zwischen den Positionen 2 und 3 des Triazinrings bzw. Dihydrotriazinrings liegt.

Bevorzugt handelt es sich bei den Dihydrotriazinverbindungen um Dihydrotriazinverbindungen mit nachfolgender Strukturformel I: 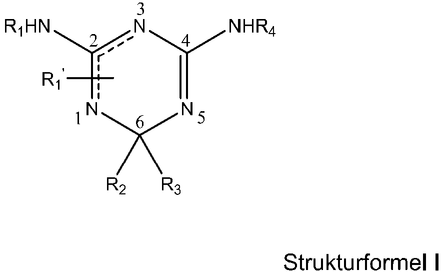 wobei
- R₁ (i) eine Phenylgruppe oder eine Phenylalkylgruppe, von denen jede optional mit 1 bis 3 Substituenten, ausgewählt aus der Gruppe bestehend aus C₁₋₆-Alkoxygruppe, Hydroxygruppe, einem Halogenatom, C₁₋₆-Halogenalkylgruppe, C₁₋₆-Alkylgruppe, einer Sulfonamidogruppe und C₁₋₆-Halogenalkoxygruppe, substituiert ist, (ii) eine Naphthylgruppe oder eine Naphthylalkylgruppe, (iii) eine heterocyclische Gruppe, eine heterocyclische Alkylgruppe oder eine heterocyclische Aminoalkylgruppe, (iv) eine Alkylgruppe mit 1 bis 16 Kohlenstoffatomen oder (v) eine Cycloalkylgruppe oder eine Cycloalkyl-alkylgruppe bedeutet,
- R₁' ein Wasserstoffatom, das an Position 1 oder 3 des Dihydrotriazinrings an das Stickstoffatom gebunden ist, bedeutet,
- R₂ und R₃ unabhängig voneinander ein Wasserstoffatom oder eine Methylgruppe bedeuten,
- R₄ eine Alkylgruppe mit 7 bis 16 Kohlenstoffatomen bedeutet und
- die Strichlinie angibt, dass die Stellung einer Doppelbindung entweder zwischen den Positionen 1 und 2 oder zwischen den Positionen 2 und 3 des Dihydrotriazinrings liegt,
Insbesondere kann es sich bei der Dihydrotriazinverbindung um ein Tautomer der Dihydrotriazinverbindung der Strukturformel I, oder ein Salz, insbesondere pharmakologisch akzeptables Salz, der Dihydrotriazinverbindung der Strukturformel I handeln.

Unter dem Ausdruck "Phenlygruppe" soll im Sinne der vorliegenden Erfindung ein Benzolrest, also die Atomgruppe -C₆H₅, verstanden werden.

Unter dem Ausdruck "Benzlygruppe" soll im Sinne der vorliegenden Erfindung die Phenylmethylgruppe -CH₂-C₆H₅, früher auch als α-Tolylgruppe bezeichnet, verstanden werden.

Der Ausdruck "Phenylalkylgruppe" bedeutet im Sinne der vorliegenden Erfindung eine Gruppe, bei welcher eine lineare, d.h. unverzweigte, oder verzweigte Alkylgruppe oder Alkylengruppe, insbesondere mit 1 bis 6 Kohlenstoffatomen, an eine Phenylgruppe gebunden ist. Die Alkylgruppe oder Alkylengruppe kann dabei eine substituierte oder unsubstituierte Alkylgruppe oder Alkylengruppe sein. Bevorzugt handelt es sich bei der Phenylalkylgruppe um eine Benzylgruppe, Methylbenzlygruppe wie insbesondere 4-Methylbenzylgruppe, 1-Phenylethylgruppe, 2-Phenylethylgruppe, 1-Phenylpropylgruppe, 2-Phenylpropylgruppe oder 3-Phenylpropylgruppe.

Ein Benzolring der Phenylgruppe oder Phenylalkylgruppe kann ein bis drei Substituenten aufweisen, insbesondere ausgewählt aus der Gruppe bestehend aus Halogenatom, Hydroxygruppe, C₁₋₆-Alkylgruppe, C₁₋₆-Haloalkylgruppe, C₁₋₆-Alkoxygruppe, C₁₋₆-Haloalkoxygruppe und Sulfonamidogruppe.

Der Ausdruck "Halogenatom" kann im Sinne der vorliegenden Erfindung ein Fluoratom, ein Chloratom, ein Bromatom oder ein lodatom bedeuten.

Der Ausdruck "C₁₋₆-Alkylgruppe" kann im Sinne der vorliegenden Erfindung eine entsprechende, d.h. eine 1 bis 6 Kohlenstoffatome aufweisende, lineare oder verzweigte Alkylgruppe bedeuten. Die Alkylgruppe kann dabei eine substituierte oder unsubstituierte Alkylgruppe sein. Beispielsweise kann es sich bei der C₁₋₆-Alkylgruppe um eine Methylgruppe, Ethylgruppe, n-Propylgruppe, Isopropylgruppe, n-Butylgruppe, Isobutylgruppe, sec-Butylgruppe, tert-Butylgruppe, n-Pentylgruppe, sec-Pentylgruppe, Isopentylgruppe, Neopentylgruppe, n-Hexylgruppe oder Isohexylgruppe handeln.

Der Ausdruck "C₁₋₆-Haloalkylgruppe" kann im Sinne der vorliegenden Erfindung insbesondere eine Chloromethylgruppe, Bromomethylgruppe, 1-Chloroethylgruppe oder Trifluoromethylgruppe bedeuten.

Der Ausdruck "C₁₋₆-Alkoxygruppe" kann im Sinne der vorliegenden Erfindung insbesondere eine Methoxygruppe, Ethoxygruppe, n-Propoxygruppe, Isopropoxygruppe, n-Butoxygruppe oder Isobutoxygruppe bedeuten.

Der Ausdruck "C₁₋₆-Haloalkoxygruppe" kann im Sinne der vorliegenden Erfindung insbesondere eine Trifluoromethoxygruppe bedeuten.

Der Ausdruck "Naphthylgruppe" kann im Sinne der vorliegenden Erfindung eine 1-Naphthylgruppe oder eine 2-Naphthylgruppe bedeuten.

Der Ausdruck "Naphthylalkylgruppe" bedeutet im Sinne der vorliegenden Erfindung eine Gruppe, bei welcher eine lineare, d.h. unverzweigte, oder verzweigte Alkylgruppe, insbesondere mit 1 bis 6 Kohlenstoffatomen, an eine Naphthylgruppe gebunden ist. Die Alkylgruppe kann dabei eine substituierte oder unsubstituierte Alkylgruppe sein. Bevorzugt handelt es sich bei der Naphthylalkylgruppe um eine 1-Naphthylmethylgruppe, 2-Naphthylmethylgruppe, 1-Naphthylethylgruppe oder 2-Naphthylethylgruppe.

Der Ausdruck "heterocyclische Gruppe" bedeutet im Sinne der vorliegenden Erfindung eine dreibis sechsgliedrige heterocyclische Gruppe, welche ein bis drei Atome, ausgewählt aus der Gruppe bestehend aus Stickstoffatom, Sauerstoffatom und Schwefelatom, enthält, wobei an die heterocyclische Gruppe ein Benzolring anelliert oder kondensiert sein kann. Die heterocyclische Gruppe kann beispielsweise eine Pyridylgruppe, eine Pyrazinylgruppe, eine Thiazolylgruppe, eine Piperidylgruppe, eine Piperazylgruppe, eine Tetrahydrofurylgruppe, eine Thienylgruppe, eine Pyrrolylgruppe, eine Pyrrolidinylgruppe, eine Oxazolylgruppe, eine Imidazolylgruppe, eine Isooxazolylgruppe, eine Isothiazolylgruppe, eine Pyrazolylgruppe, eine Tetrahydropyranylgruppe, eine 2-Oxotetrahydropyranylgruppe, eine Pyrimidinylgruppe, eine Pyradizinylgruppe, eine Morpholinylgruppe, eine 1,3,5-Triazinylgruppe, eine 1,2,4-Triazinylgruppe, eine Quinolylgruppe oder eine Isoquinolylgruppe bedeuten.

Insbesondere kann die heterocyclische Gruppe im Sinne der vorliegenden Erfindung eine 2-Pyridylgruppe, eine 3-Pyridylgruppe, eine 4-Pyridylgruppe, eine 2-Furylgruppe, eine 2-Thiazolylgruppe, eine 1-Piperidylgruppe, eine 1-Piperazylgruppe, eine 2-Quinolylgruppe, eine 3-Quinolylgruppe, eine 4-Quinolylgruppe, eine 5-Quinolylgruppe, eine 8-Quinolylgruppe, eine 1-Isoquinolylgruppe, eine 3-Isoquinolylgruppe, eine 4-Isoquinolylgruppe oder eine 5-Isoquinolylgruppe bedeuten.

Der Ausdruck "heterocyclische Alkylgruppe" bedeutet im Sinne der vorliegenden Erfindung eine Gruppe, bei welcher eine lineare, d.h. unverzweigte, oder verzweigte Alkylgruppe, insbesondere mit 1 bis 6 Kohlenstoffatomen, an eine heterocyclische Gruppe, insbesondere wie in den vorherigen Absätzen definiert oder beschrieben, gebunden ist. Die Alkylgruppe kann dabei eine substituierte oder unsubstituierte Alkylgruppe sein. Bevorzugt handelt es sich bei der heterocyclischen Alkylgruppe um eine 2-Pyridylmethylgruppe, 3-Pyridylmethylgruppe, 4-Pyridylmethylgruppe, 2-Pyridylethylgruppe, 3-Pyridylethylgruppe, 4-Pyridylethylgruppe, Pyrazinylmethylgruppe, Pyrazinylethylgruppe, 2-Furylmethylgruppe, 2-Furylethylgruppe, 2-Thiazolylmethylgruppe, 2-Thiazolylethylgruppe, 4-Piperidylmethylgruppe, 2-Quinolylmethylgruppe, 3-Quinolylmethylgruppe, 4-Quinolylmethylgruppe, 5-Quinolylmethylgruppe, 8-Quinolylmethylgruppe, 1-Isoquinolylmethylgruppe, 3-Isoquinolylmethylgruppe, 4-Isoquinolylmethylgruppe oder 5-Isoquinolylmethylgruppe.

Der Ausdruck "heterocyclische Aminoalkylgruppe" bedeutet im Sinne der vorliegenden Erfindung eine Gruppe, bei welcher eine lineare, d.h. unverzweigte, oder verzweigte Alkylgruppe, insbesondere mit 1 bis 12 Kohlenstoffatomen, an eine heterocyclische Aminogruppe gebunden ist. Die Alkylgruppe kann dabei eine substituierte oder unsubstituierte Alkylgruppe sein. Bevorzugt handelt es sich bei der heterocyclischen Aminoalkylgruppe um eine 4-Amino-dihydro-1,3,5-triazin-2-yl-aminogruppe, eine 4-Alkylamino-dihydro-1,3,5-triazin-2-yl-aminogruppe oder 4-Phenylalkylamino-dihydro-1,3,5-triazin-2-yl-aminogruppe.

Der Ausdruck "Alkylgruppe mit 1 bis 16 Kohlenstoffatomen" bedeutet im Sinne der vorliegenden Erfindung eine lineare, d.h. unverzweigte, oder verzweigte Alkylgruppe mit 1 bis 16 Kohlenstoffatomen. Die Alkylgruppe kann dabei eine substituierte oder unsubstituierte Alkylgruppe sein. Bevorzugt handelt es sich bei der Alkylgruppe mit 1 bis 16 Kohlenstoffatomen um eine Methylgruppe, Ethylgruppe, n-Propylgruppe, Isopropylgruppe, n-Butylgruppe, Isobutylgruppe, sec-Butylgruppe, tert-Butylgruppe, n-Hexylgruppe, n-Heptylgruppe, n-Octylgruppe, tert-Octylgruppe, n-Nonylgruppe, n-Decylgruppe, n-Undecylgruppe, n-Dodecylgruppe, n-Tridecylgruppe, n-Tetradecylgruppe, n-Pentadecylgruppe oder n-Hexadecylgruppe.

Der Ausdruck "Cycloalkylgruppe" bedeutet im Sinne der vorliegenden Erfindung insbesondere eine Cycloalkylgruppe mit 3 bis 6 Kohlenstoffatomen. Beispielsweise kann es sich bei der Cycloalkylgruppe um eine Cyclopropylgruppe, Cyclobutylgruppe, Cyclopentylgruppe oder Cyclohexylgruppe handeln.

Der Ausdruck "Cycloalkyl-alkylgruppe" bedeutet im Sinne der vorliegenden Erfindung insbesondere eine Gruppe, bei welcher eine lineare, d.h. unverzweigte, oder verzweigte Alkylgruppe, insbesondere mit 1 bis 6 Kohlenstoffatomen, an eine Cycloalkylgruppe, insbesondere wie im vorherigen Absatz definiert oder beschrieben, gebunden ist. Die Alkylgruppe kann dabei eine substituierte oder unsubstituierte Alkylgruppe sein. Bevorzugt handelt es sich bei der Cycloalkyl-alkylgruppe um eine Cyclohexylmethylgruppe, eine 1-Cyclohexylethylgruppe oder eine 2-Cyclohexylethylgruppe.

Der Ausdruck "Alkylgruppe mit 7 bis 16 Kohlenstoffatomen" bedeutet im Sinne der vorliegenden Erfindung eine lineare, d.h. unverzweigte, oder verzweigte Alkylgruppe mit 7 bis 16 Kohlenstoffatomen. Die Alkylgruppe kann dabei eine substituierte oder unsubstituierte Alkylgruppe sein. Bevorzugt handelt es sich bei der Alkylgruppe mit 7 bis 16 Kohlenstoffatomen um eine n-Heptylgruppe, n-Octylgruppe, tert-Octylgruppe, n-Nonylgruppe, n-Decylgruppe, n-Undecylgruppe, n-Dodecylgruppe, n-Tridecylgruppe, n-Tetradecylgruppe, n-Pentadecylgruppe oder n-Hexadecylgruppe.

Der im Zusammenhang der Dihydrotriazinverbindung der Strukturformel I verwendete Ausdruck "Salz" kann im Sinne der vorliegenden Erfindung insbesondere ein Salz mit einer organischen Säure, wie beispielsweise Ameisensäure, Essigsäure, Propionsäure, Milchsäure, Buttersäure, Isobuttersäure, Äpfelsäure, Maleinsäure, Malonsäure, Fumarsäure, Bernsteinsäure, Bernsteinsäuremonoamid, Glutarsäure, Weinsäure, Oxalsäure, Citronensäure, Glykolsäure, Glucuronsäure, Ascorbinsäure, Asparaginsäure, Glutaminsäure, Benzoesäure, Phthalsäure, Salicylsäure, Anthranilsäure, Benzolsulfonsäure, p-Toluolsulfonsäure oder Methansulfonsäure, bedeuten.

Bevorzugt bedeutet/bedeuten in der Strukturformel I
- R₁ eine Phenylgruppe oder eine Phenylalkylgruppe von denen jede optional durch 1 bis 3 Substituenten, ausgewählt aus der Gruppe bestehend aus Fluoratom, Chloratom, Hydroxygruppe, Methylgruppe, tert-Butylgruppe, Trifluoromethylgruppe und Methoxygruppe, substituiert ist,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

Bevorzugt bedeutet/bedeuten in der Strukturformel I
- R₁ eine Phenylgruppe, eine Benzylgruppe oder eine 2-Phenylethylgruppe, von denen jede optional durch 1 bis 3 Substituenten, ausgewählt aus der Gruppe bestehend aus Fluoratom, Chloratom, Hydroxygruppe, Methylgruppe, tert-Butylgruppe, Trifluoromethylgruppe und Methoxygruppe, substituiert ist,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

Besonders bevorzugt bedeutet/bedeuten in der Strukturformel I
- R₁ eine Benzylgruppe, welche optional durch 1 bis 3 Substituenten, ausgewählt aus der Gruppe bestehend aus Fluoratom, Chloratom, Hydroxygruppe, Methylgruppe, tert-Butylgruppe, Trifluoromethylgruppe und Methoxygruppe, bevorzugt durch 1 bis 3 Methylgruppen, besonders bevorzugt durch eine Methylgruppe, substituiert ist,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

Weiter bevorzugt bedeutet/bedeuten in der Strukturformel I
- R₁ eine Phenylgruppe, 4-Chlorophenylgruppe, 2,4-Difluorophenylgruppe, 2,3,4-Trifluorophenylgruppe, 4-tert-Butylphenylgruppe, 4-Methoxyphenylgruppe, 2-Methoxy-4-tert-butylphenylgruppe, 4-Trifluoromethoxyphenylgruppe, Benzylgruppe, Methylbenzlygruppe wie insbesondere 4-Methylbenzylgruppe, 4-Methoxybenzylgruppe, 3,4-Dimethoxybenzylgruppe, 4-Hydroxybenzylgruppe, 3,4-Dichlorobenzylgruppe, 2,3,4-Trichlorobenzylgruppe, 4-Trifluoromethylbenzylgruppe, 1-Phenylethylgruppe, 2-Phenylethylgruppe, 1-Phenylpropylgruppe, 2-Phenylpropylgruppe oder 3-Phenylpropylgruppe, bevorzugt eine Methylbenzylgruppe, besonders bevorzugt eine 4-Methylbenzylgruppe,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

Insbesondere bevorzugt bedeutet/bedeuten in der Strukturformel I
- R₁ eine Methylbenzylgruppe, vorzugsweise 4-Methylbenzylgruppe, und/oder R₂ und R₃ jeweils eine Methylgruppe und/oder
- R₄ eine n-Octylgruppe.

Vorzugsweise bevorzugt bedeutet/bedeuten in der Strukturformel I
- R₁ eine Methylbenzylgruppe, vorzugsweise 4-Methylbenzylgruppe,
- R₂ und R₃ jeweils eine Methylgruppe und
- R₄ eine n-Octylgruppe.

Weiter bevorzugt bedeutet/bedeuten in der Strukturformel I
- R₁ eine Methylbenzylgruppe, vorzugsweise 4-Methylbenzylgruppe,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe.

Besonders bevorzugt handelt es sich bei der Dihydrotriazinverbindung um 4-Octylamino-1,6-dihydro-6,6-dimethyl-2-(4'-methylbenzylamino)-1,3,5-triazingluconat, insbesondere 4-Octylamino-1,6-dihydro-6,6-dimethyl-2-(4'-methylbenzylamino)-1,3,5-triazin-D-gluconat. Alternativ oder in Kombination kann es sich bei der Dihydrotriazinverbindnung um 4-Octylamino-3,6-dihydro-6,6-dimethyl-2-(4'-methylbenzylamino)-1,3,5-triazingluconat, insbesondere 4-Octylamino-3,6-dihydro-6,6-dimethyl-2-(4'-methylbenzylamino)-1,3,5-triazin-D-gluconat, handeln.

Der antimikrobielle Wirkstoff der erfindungsgemäßen Zusammensetzung kann einen Anteil von 0,001 Gew.-% bis 1 Gew.-%, insbesondere 0,01 Gew.-% bis 0,5 Gew.-%, vorzugsweise 0,025 Gew.-% bis 0,25 Gew.-%, aufweisen, bezogen auf das Gesamtgewicht der Zusammensetzung.

In weiterer Ausgestaltung der Erfindung weist die Zusammensetzung ferner ein Tensid auf. Bei dem Tensid handelt es sich vorzugsweise um ein zwitterionisches Tensid, insbesondere Betain-Tensid.

Unter dem Ausdruck "zwitterionisches Tensid" soll im Sinne der vorliegenden Erfindung ein Tensid verstanden werden, das sowohl eine negativ als auch eine positiv geladene funktionelle Gruppe besitzt. Wie jedes Tensid ist auch ein zwitterionisches Tensid aus einem polaren und einem unpolaren Teil aufgebaut. Als unpolarer Teil dient eine Alkylgruppe, als polarer Teil meist eine Carboxylat-Gruppe (R-COO-) zusammen mit einer quartären Ammonium-Gruppe (R₄N+).

Unter dem Ausdruck "Betain-Tensid" soll im Sinne der vorliegenden Erfindung ein zwitterionisches Tensid mit einer Betain-Struktur oder einer von Betain abgeleiteten Struktur verstanden werden.

Vorzugsweise weist das Betain-Tensid die nachfolgende Strukturformel II auf: 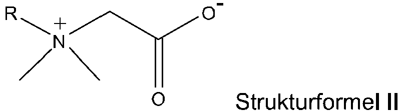 wobei R ein organischer Rest bedeutet.

Bevorzugt ist das zwitterionische Tensid oder Betain-Tensid ein Alkylamidoethylbetain, ein Alkylamidopropylbetain oder eine Mischung davon.

Bei dem Alkylamidoalkylbetain handelt es sich besonders bevorzugt um ein Alkylamidoalkylbetain einer Fettsäure. Bevorzugt weist die Fettsäure dabei 8 bis 18 Kohlenstoffatome auf. Bei der Fettsäure kann es sich um eine gesättigte oder ungesättigte Fettsäure handeln. Bevorzugt handelt es sich bei der Fettsäure um Caprylsäure, Caprinsäure, Undecylensäure (Undec-10-ensäure), Undecylsäure (n-Undecansäure), Laurinsäure, Stearinsäure, Ricinolsäure oder Kokosfettsäure.

Bevorzugt ist das Alkylamidoalkylbetain ausgewählt aus der Gruppe bestehend aus Caprylamidoalkylbetain, Caprinamidoalkylbetain, Undecylenamidoalkylbetain, Undecylamido-alkylbetain, Lauramidoalkylbetain, Lauryldimethylaminoessigsäurebetain, Stearinamidoalkyl-betain, Ricinolamidoalkylbetain, Cocamidoalkylbetain und Mischungen von wenigstens zwei der vorgenannten Alkylamidoalkylbetaine.

Das Alkylamidoethylbetain ist bevorzugt ein Alkylamidoethylbetain auf Basis einer Fettsäure, insbesondere einer Fettsäure mit 8 bis 18 Kohlenstoffatomen. Bei der Fettsäure kann es sich um eine gesättigte oder ungesättigte Fettsäure handeln. Bevorzugt handelt es sich bei der Fettsäure um Caprylsäure, Caprinsäure, Undecylensäure (Undec-10-ensäure), Undecylsäure (n-Undecansäure), Laurinsäure, Stearinsäure, Ricinolsäure oder Kokosfettsäure.

Besonders bevorzugt ist das Alkylamidoethylbetain ausgewählt aus der Gruppe bestehend aus Caprylamidoethylbetain, Caprinamidoethylbetain, Undecylenamidoethylbetain, Undecylamidoethylbetain, Lauramidoethylbetain, Cocamidoethylbetain, Stearinamidoethylbetain, Ricinolamidoethylbetain und Kombinationen, insbesondere Mischungen, von wenigstens zwei der vorgenannten Alkylamidoethylbetaine.

Bei dem Alkylamidopropylbetain handelt es sich bevorzugt um ein Alkylamidopropylbetain auf Basis einer Fettsäure, insbesondere einer Fettsäure mit 8 bis 18 Kohlenstoffatomen. Bei der Fettsäure kann es sich um eine gesättigte oder ungesättigte Fettsäure handeln. Bevorzugt handelt es sich bei der Fettsäure um Caprylsäure, Caprinsäure, Undecylensäure (Undec-10-ensäure), Undecylsäure (n-Undecansäure), Laurinsäure, Stearinsäure, Ricinolsäure oder Kokosfettsäure.

Besonders bevorzugt ist das Alkylamidopropylbetain ausgewählt aus der Gruppe bestehend aus Caprylamidopropylbetain, Caprinamidopropylbetain, Undecylenamidopropylbetain, Undecylamidopropylbetain, Cocamidopropylbetain, Stearinamidopropylbetain, Ricinolamidopropylbetain und Kombinationen, insbesondere Mischungen, von wenigstens zwei der vorgenannten Alkylamidopropylbetaine.

Alternativ oder in Kombination kann es sich bei dem Tensid um ein nichtionisches Tensid handeln.

Unter dem Ausdruck "nichtionisches Tensid" soll im Sinne der vorliegenden Erfindung ein Tensid bezeichnet werden, welches keine dissoziierbaren funktionellen Gruppen enthält und sich daher in Wasser oder einer wässrigen Flüssigkeit nicht in Ionen auftrennt.

Das nichtionische Tensid ist vorzugsweise ausgewählt aus der Gruppe bestehend aus Poloxamer, Fettalkoholalkoxylat wie Fettalkoholethoxylat, Polyvinylpyrrolidon, Alkylpolyglucosid und Mischungen von wenigstens zwei der vorgenannten nichtionischen Tenside.

Bevorzugt weist das Poloxamer 2 bis 130 Struktureinheiten -CH2-CH2-O- und/oder 15 bis 67 Struktureinheiten -CHCH3-CH2-O- pro Molekül auf.

Bei dem Poloxamer kann es sich insbesondere um Poloxamer 407, Poloxamer 188 oder um eine Kombination, insbesondere Mischung, davon handeln.

Unter dem Ausdruck "Fettalkoholalkoxylat" soll im Sinne der vorliegenden Erfindung ein nichtionisches Tensid verstanden werden, dessen lipophiler Teil einen Fettalkohol aufweist oder aus einem Fettalkohol besteht und dessen hydrophiler Teil ein Polyalkylenglykol, insbesondere kurzkettiges Polyalkylenglykol, aufweist oder aus einem Polyalkylenglykol, insbesondere kurzkettigen Polyalkylenglykol, besteht. Bei dem Fettalkohol kann es sich insbesondere um einen von Capryl-, Caprin-, Laurin-, Palmitin-, Stearin- oder Ölsäure abgeleiteten Alkohol oder um verzweigte Isononyl-, Isoundecyl-, Isotridecyl-, Isopentadecyl- oder Isononadecylalkohole handeln. Das Fettalkoholalkoxylat kann im Sinne der vorliegenden Erfindung auch als Polyalkylenglykolether bezeichnet werden.

Wie bereits erwähnt, kann es sich bei dem Fettalkoholalkoxylat insbesondere um ein Fettalkoholethoxylat handeln.

Bevorzugt handelt es sich bei dem Fettalkoholethoxylat um ein Polyoxyethylenether des Laurylalkohols, ein Polyoxyethylenether des Myristylalkohols, ein Polyoxyethylenether des Cetylalkohols, ein Polyoxyethylenether des Cetylstearylalkohols, ein Polyoxyethylenether des Stearylalkohols, ein Polyoxyethylenether des Oleylalkohols, ein Polyoxyethylenether des Isononanalkohols, ein Polyoxyethylenether des Isoundecanalkohols, ein Polyoxyethylenether des Isotridecanalkohols, ein Polyoxyethylenether des Isopentadecanalkohols, ein Polyoxyethylenether des Isoheptadecanalkohols, ein Polyoxyethylenether des Isononadecanalkohols oder um eine Mischung von wenigstens zwei der vorgenannten Polyoxyethylenether.

Das Fettalkoholethoxylat kann insbesondere ausgewählt sein aus der Gruppe bestehend aus Polxoxyethylen (4) laurylether, Polxoxyethylen (7) laurylether, Polyoxyethylen (9) laurylether, Polyoxyethylen (23) laurylether, Polyoxyethylen (2) cetylether, Polyoxyethylen (10) cetylether, Polyoxyethylen (20) cetylether, Polyoxyethylen (6) cetylstearylether, Polyoxyethylen (20) cetylstearylether, Polyoxyethylen (25) cetylstearylether, Polyoxyethylen (2) stearylether, Polyoxyethylen (10) stearylether, Polyoxyethylen (20) stearylether, Polyoxyethylen (2) oleylether, Polyoxyethylen (10) oleylether, Polyoxyethylen (20) oleylether, Polyoxyethylen (10) monodecylether, Polyoxyethylen (10) tridecylether und Mischungen von wenigstens zwei der vorgenannten Fettalkoholethoxylate.

Mit anderen Worten kann das Fettalkoholethoxylat insbesondere ausgewählt sein aus der Gruppe bestehend aus Laureth-4, Laureth-7, Laureth-9, Laureth-23, Ceteth-2, Ceteth-10, Ceteth-20, Ceteareth-6, Ceteareth-20, Ceteareth-25, Steareth-2, Steareth-10, Steareth-20, Oleth-2, Oleth-10, Oleth-20, Deceth-10, Trideceth-10 und Mischungen von wenigstens zwei der vorgenannten Fettalkoholethoxylate.

Unter dem Ausdruck "Alkylpolyglucosid" soll im Sinne der vorliegenden Erfindung ein nichtionisches Zuckertensid verstanden werden, welches einen oder mehrere Glucosebausteine und einen Alkylrest, insbesondere langkettigen Alkylrest, aufweist oder aus einem oder mehreren Glucosebausteinen und einem Alkylrest, insbesondere langkettigen Alkylrest, besteht. Der Glucosebaustein bzw. die Glucosebausteine fungieren als hydrophiler Bestandteil, während der Alkylrest die hydrophobe Gruppe darstellt.

Das Alkylpolyglucosid weist vorzugsweise 1 bis 5 Glucosebausteine und/oder einen Alkylrest mit 6 Kohlenstoffatomen bis 20 Kohlenstoffatomen, insbesondere 6 Kohlenstoffatomen bis 16 Kohlenstoffatomen, bevorzugt 8 Kohlenstoffatomen bis 14 Kohlenstoffatomen, auf.

Bevorzugt handelt es sich bei dem Alkylpolyglucosid um eine C8- bis C20-Alkylpolyglucose, insbesondere C8- bis C16-Alkylpolyglucose.

Besonders bevorzugt handelt es sich bei dem Alkylpolyglucosid um Laurylpolyglucose, Decylpolyglucose, Cocoylpolyglucose oder um eine Mischung von wenigstens zwei der vorgenannten Alkylpolyglucoside. Der Alkylrest der Decylpolyglucose weist dabei bevorzugt 8 Kohlenstoffatome bis 16 Kohlenstoffatome, insbesondere 10 Kohlenstoffatome, auf. Der Alkylrest der Laurylpolyglucose weist bevorzugt 12 Kohlenstoffatome bis 16 Kohlenstoffatome, insbesondere 12 Kohlenstoffatome, auf. Der Alkylrest der Cocoylpolyglucose weist bevorzugt 8 Kohlenstoffatome bis 16 Kohlenstoffatome auf.

Das Tensid der erfindungsgemäßen Zusammensetzung kann einen Anteil von 0,01 Gew.-% bis 10 Gew.-%, insbesondere 0,05 Gew.-% bis 2 Gew.-%, vorzugsweise 0,1 Gew.-% bis 1 Gew.-%, aufweisen, bezogen auf das Gesamtgewicht der Zusammensetzung.

In weiterer Ausgestaltung der Erfindung weist die Zusammensetzung ferner ein Feuchthaltemittel auf. Das Feuchthaltemittel, das im Sinne der vorliegenden Erfindung auch als feuchtigkeitsspendende Substanz bezeichnet werden kann, kann insbesondere ein Polyalkohol sein. Bevorzugt ist das Feuchthaltemittel ausgewählt aus der Gruppe bestehend aus Aloe Barbadensis-Extrakt, Glycerin, Polydextrose, Sorbit, Ethylenglykol, Polyethylenglykol, Propylenglykol, Butylenglykol, Pentylenglykol, Hexandiol, Octandiol, Glucose, Fructose, Glucuronsäure, Lactose, Milchsäure, Lactat, Lactulose, Saccharose, Hyaluronsäure, Xylitol, Xylose und Mischungen von wenigstens zwei der vorgenannten Feuchthaltemittel. Durch ein Feuchthaltemittel können der Zusammensetzung vorteilhaftweise, insbesondere zusätzliche, feuchtigkeitsspendende Eigenschaften verliehen werden, wodurch der Wundheilungsverlauf ebenfalls günstig beeinflusst werden kann.

Das Feuchthaltemittel kann einen Anteil von 0,05 Gew.-% bis 20 Gew.-%, insbesondere 0,075 Gew.-% bis 17,5 Gew.-%, vorzugsweise 0,1 Gew.-% bis 15 Gew.-%, aufweisen, bezogen auf das Gesamtgewicht der Zusammensetzung.

In weiterer Ausgestaltung der Erfindung weist die Zusammensetzung ferner ein Verdickungsmittel, insbesondere ein Geliermittel, auf. Das Verdickungsmittel kann insbesondere ausgewählt sein aus der Gruppe bestehend aus Celluloseether, Hydroxyethylcellulose, Carboxymethylcellulose, Hydroxypropylcellulose, Hydroxypropylmethylcellulose, Ethylhydroxyethylcellulose, Methylcellulose, Ethylcellulose, Methylethylcellulose, Natriumcarboxymethylcellulose, Chitosan, Xanthan, Stärke, Gelatine, Casein und Mischungen von wenigstens zwei der vorgenannten Verdickungsmittel. Durch die Verwendung eines Verdickungsmittel kann die Zusammensetzung in einer zähflüssigen oder viskosen Form, insbesondere in Form eines Hydrogels oder einer Creme, bereitgestellt werden, was insbesondere im Hinblick auf eine topische, d.h. lokale, Verabreichung der Zusammensetzung von Vorteil ist.

Das Verdickungsmittel kann einen Anteil von 0,01 Gew.-% bis 10 Gew.-%, insbesondere 0,1 Gew.-% bis 7,5 Gew.-%, vorzugsweise 1 Gew.-% bis 5 Gew.-%, aufweisen, bezogen auf das Gesamtgewicht der Zusammensetzung.

Ferner kann die Zusammensetzung zusätzlich einen Komplexbildner aufweisen. Der Komplexbildner kann insbesondere ausgewählt sein aus der Gruppe bestehend aus Citronensäure, Weinsäure, Bernsteinsäure, Methylglycindiacetat, Ethyldiamintetraacetat (EDTA), N-(2-Hydroxyethyl)ethylendiamin-triessigsäure Trinatriumsalz, N,N'-Bis-(carboxymethyl)-L-glutamat, Polyasparaginsäure, Iminodisuccinat, Salze der vorgenannten Komplexbildner und Mischungen von wenigstens zwei der vorgenannten Komplexbildner.

Der Komplexbildner kann einen Anteil von 0,001 Gew.-% bis 0,5 Gew.-%, insbesondere 0,005 Gew.-% bis 0,25 Gew.-%, vorzugsweise 0,01 Gew.-% bis 0,1 Gew.-%, aufweisen, bezogen auf das Gesamtgewicht der wässrigen Zusammensetzung.

Ferner kann die Zusammensetzung zusätzlich einen pH-Regulator aufweisen. Der pH-Regulator kann ausgewählt sein aus der Gruppe bestehend aus Calciumcarbonat, Magnesiumhydroxid, Natronlauge, Natriumhydrogencarbonat, Triethanolamin, Salzsäure, Essigsäure, Acetat, Milchsäure, Äpfelsäure, Fumarsäure, Lactat, Zitronensäure, Citrat, Weinsäure, Tartrat, Malat, Gluconsäure, Gluconat, Glykolsäure, Glykolat und Mischungen von wenigstens zwei der vorgenannten pH-Regulatoren.

Der pH-Regulator kann einen Anteil von 0,001 Gew.-% bis 50 Gew.-%, insbesondere 0,005 Gew.- % bis 25 Gew.-%, vorzugsweise 0,01 Gew.-% bis 10 Gew.-%, aufweisen, bezogen auf das Gesamtgewicht der Zusammensetzung.

Ferner kann die Zusammensetzung zusätzlich einen Stabilisator, insbesondere ein Antioxidans, aufweisen. Der Stabilisator kann insbesondere ausgewählt sein aus der Gruppe bestehend aus Ascorbinsäure (Vitamin C), Tocopherole, Tocotrienole, polyphenolische Antioxidantien wie Resveratrol und/oder Flavonoide, Carotinoide wie Lycopin und/oder Betacarotin und/oder Lutein, Galate, Butylhydroxyanisol, Butylhydroxytoluol, Ascorbylpalmitat, Ascorbylstearat, Tocopherolacetat und Mischungen von wenigstens zwei der vorgenannten Stabilisatoren.

Der Stabilisator kann einen Anteil von 0,001 Gew.-% bis 0,5 Gew.-%, insbesondere 0,005 Gew.- % bis 0,25 Gew.-%, vorzugsweise 0,01 Gew.-% bis 0,1 Gew.-%, aufweisen, bezogen auf das Gesamtgewicht der Zusammensetzung.

Ferner kann die Zusammensetzung zusätzlich ein Vitamin und/oder ein Provitamin, d.h. eine Vorstufe eines Vitamins, aufweisen. Das Vitamin kann insbesondere ausgewählt sein aus der Gruppe bestehend aus Ascorbinsäure (Vitamin C), Tocopherole, Tocotrienole, D-Panthenol und Mischungen von wenigstens zwei der vorgenannten Vitamine. Das Provitamin kann insbesondere ausgewählt sein aus der Gruppe bestehend aus Provitamin von Ascorbinsäure (Vitamin C), Provitamine von Tocopherole, Provitamine von Tocotrienole, Provitamin von D-Panthenol und Mischungen von wenigstens zwei der vorgenannten Provitamine.

Das Vitamin und/oder Provitamin können/kann einen Anteil von 0,001 Gew.-% bis 0,5 Gew.-%, insbesondere 0,005 Gew.-% bis 0,25 Gew.-%, vorzugsweise 0,01 Gew.-% bis 0,1 Gew.-%, aufweisen, bezogen auf das Gesamtgewicht der Zusammensetzung.

Ferner kann die Zusammensetzung zusätzlich ein Salz, insbesondere ein antimikrobielles, insbesondere antibakterielles, und/oder die Osmolarität beeinflussendes oder regulierendes Salz, aufweisen. Das Salz kann beispielsweise ausgewählt sein aus der Gruppe bestehend aus Eisensalze, Zinksalze, Natriumchlorid, Kaliumchlorid, Calciumchlorid, Magnesiumchlorid und Mischungen von wenigstens zwei der vorgenannten Salze.

In weiterer Ausgestaltung der Erfindung ist die Zusammensetzung eine wässrige, d.h. Wasser aufweisende, Zusammensetzung. Insbesondere kann die Zusammensetzung einen Wasseranteil von 60 Gew.-% bis 99 Gew.-%, insbesondere 70 Gew.-% bis 95 Gew.-%, vorzugsweise 80 Gew.- % bis 90 Gew.-%, aufweisen, bezogen auf das Gesamtgewicht der Zusammensetzung.

In weiterer Ausgestaltung der Erfindung weist die Zusammensetzung Folgendes auf:
- L-Arginin und/oder ein Salz von L-Arginin,
- einen antimikrobiellen Wirkstoff, vorzugsweise Polyhexanid,
- ein Betain-Tensid,
- ein Feuchthaltemittel und
- Wasser.

Bezüglich des L-Arginins und/oder Salzes von L-Arginin, des antimikrobiellen Wirkstoffs, des Betain-Tensids sowie des Feuchthaltemittels wird vollumfänglich auf die bisherige Beschreibung Bezug genommen. Die dort in Bezug auf das L-Arginin, den antimikrobiellen Wirkstoff, das Betain-Tensid, das Feuchthaltemittel sowie das Wasser beschriebenen Merkmale und Vorteile gelten auch für diese Ausgestaltung der Erfindung.

In weiterer Ausgestaltung der Erfindung weist die Zusammensetzung, bezogen auf das Gesamtgewicht der Zusammensetzung, Folgendes auf:
- 5 Gew.-% bis 15 Gew.-% von L-Arginin und/oder eines Salzes von L-Arginin,
- 0,025 Gew.-% bis 0,25 Gew.-% eines antimikrobiellen Wirkstoffs, vorzugsweise Polyhexanid,
- 0,1 Gew.-% bis 1 Gew.-% eines Betain-Tensids,
- 0,1 Gew.-% bis 15 Gew.-% eines Feuchthaltemittels und
- Rest Wasser.

Bezüglich des L-Arginins und/oder Salzes von L-Arginin, des antimikrobiellen Wirkstoffs, des Betain-Tensids sowie des Feuchthaltemittels wird ebenfalls vollumfänglich auf die bisherige Beschreibung Bezug genommen. Die dort in Bezug auf das L-Arginin, den antimikrobiellen Wirkstoff, das Betain-Tensid, das Feuchthaltemittel sowie das Wasser beschriebenen Merkmale und Vorteile gelten auch für diese Ausgestaltung der Erfindung.

In weiterer Ausgestaltung der Erfindung weist die Zusammensetzung Folgendes auf:
- L-Arginin und/oder ein Salz von L-Arginin,
- einen antimikrobiellen Wirkstoff, vorzugsweise Polyhexanid,
- ein Betain-Tensid,
- ein Feuchthaltemittel,
- ein Verdickungsmittel und
- Wasser.

Bezüglich des L-Arginins und/oder Salzes von L-Arginin, des antimikrobiellen Wirkstoffs, des Betain-Tensids, des Feuchthaltemittels sowie des Verdickungsmittels wird ebenfalls vollumfänglich auf die bisherige Beschreibung Bezug genommen. Die dort in Bezug auf das L-Arginin, den antimikrobiellen Wirkstoff, das Betain-Tensid, das Feuchthaltemittel, das Verdickungsmittel sowie das Wasser beschriebenen Merkmale und Vorteile gelten auch für diese Ausgestaltung der Erfindung.

In weiterer Ausgestaltung der Erfindung weist die Zusammensetzung, bezogen auf das Gesamtgewicht der Zusammensetzung, Folgendes auf:
- 5 Gew.-% bis 15 Gew.-% von L-Arginin und/oder eines Salzes von L-Arginin,
- 0,025 Gew.-% bis 0,25 Gew.-% eines antimikrobiellen Wirkstoffs, vorzugsweise Polyhexanid,
- 0,1 Gew.-% bis 1 Gew.-% eines Betain-Tensids,
- 0,1 Gew.-% bis 15 Gew.-% eines Feuchthaltemittels,
- 1 Gew.-% bis 5 Gew.-% eines Verdickungsmittels und
- Rest Wasser.

Bezüglich des L-Arginins und/oder Salzes von L-Arginin, des antimikrobiellen Wirkstoffs, des Betain-Tensids, des Feuchthaltemittels sowie des Verdickungsmittels wird ebenfalls vollumfänglich auf die bisherige Beschreibung Bezug genommen. Die dort in Bezug auf das L-Arginin, den antimikrobiellen Wirkstoff, das Betain-Tensid, das Feuchthaltemittel, das Verdickungsmittel sowie das Wasser beschriebenen Merkmale und Vorteile gelten auch für diese Ausgestaltung der Erfindung.

Die Erfindung basiert ferner auf der überraschenden Erkenntnis, dass insbesondere eine Zusammensetzung gemäß den vier zuletzt beschriebenen Ausgestaltungen der Erfindung aufgrund ihres multifunktionalen Charakters sowie insbesondere aufgrund synergistischer Effekte durch das Zusammenspiel von L-Arginin und/oder des Salzes von L-Arginin, des antimikrobiellen Wirkstoffs, des Betain-Tensids sowie des Feuchthaltemittels bzw. von L-Arginin und/oder des Salzes von L-Arginin, des antimikrobiellen Wirkstoffs, des Betain-Tensids, des Feuchthaltemittels sowie des Verdickungsmittels besonders wirkungsvoll bei der Behandlung von Wunden ist. Dies ist vor allem im Hinblick auf die Behandlung von chronischen Wunden, die oft schwer heilbar sind und eine insbesondere komplexe Therapie erforderlich machen, von Vorteil.

In weiterer Ausgestaltung der Erfindung liegt die Zusammensetzung in Form eines Hydrogels vor. Bei dieser Ausgestaltung der Erfindung kann die Zusammensetzung insbesondere eine Viskosität von 1000 mPas bis 45000 mPas, insbesondere 3000 mPas bis 35000 mPas, vorzugsweise 5000 mPas bis 25000 mPas, aufweisen. Die Viskositätsmessung wird ohne Verdünnung bei einer Drehzahl von 84 rpm, einer Messdauer von 50 s und einer Temperatur von 25 °C durchgeführt. Dabei kann die Viskositätsmessung insbesondere mit einer RV7-Spindel durchgeführt werden.

Alternativ kann die Zusammensetzung in Form einer wässrigen Flüssigkeit, insbesondere wässrigen Lösung, oder Creme vorliegen.

In weiterer Ausgestaltung der Erfindung ist die Zusammensetzung für eine topische, d.h. lokale bzw. nichtsystemische, Verabreichung hergerichtet. Durch eine lokale Verabreichung der Zusammensetzung können vorteilhafterweise unerwünschte Nebenwirkungen oder Kontraindikationen, wie sie bei einer systemischen Verabreichung auftreten können, vermieden werden. Dadurch lässt sich die Zusammensetzung auch bei Patienten verabreichen, bei welchen eine Verabreichung von L-Arginin und/oder eines Salzes von L-Arginin ansonsten kontraindiziert wäre. Beispielsweise lassen sich mithilfe der erfindungsgemäßen Zusammensetzung auch Wunden von Patienten mit Herz-Kreislauf-Erkrankungen oder einer blutdrucksenkenden, blutverdünnenden oder potenzsteigernden Medikation behandeln. Dies eröffnet vorteilhafterweise einen breiten medizinischen Anwendungsbereich der Zusammensetzung.

Vorzugsweise ist die Zusammensetzung für eine, insbesondere unmittelbare, Verabreichung auf die Wunden hergerichtet.

Weiter kann die Zusammensetzung iso-osmolar sein, d.h. eine gleiche Osmolarität wie Blut aufweisen.

Alternativ kann die Zusammensetzung hyperosmolar sein, d.h. eine höhere Osmolarität als Blut aufweisen.

In weiterer Ausgestaltung der Erfindung handelt es sich bei den Wunden um chronische Wunden. Bei chronischen Wunden verlangsamt sich die Wundheilung bis hin zur Stagnation. Für den Betroffenen bedeutet eine chronische Wunde, neben körperlichen Beschwerden durch Schmerzen, Exsudation und auftragende Verbände, eine Einschränkung seiner Lebensqualität durch Kosten für Material und Versorgung, Abhängigkeit von anderen, Schwierigkeiten bei der Kleiderauswahl und teilweisen Verlust des selbstbestimmten Alltags. Die chronischen Wunden können insbesondere ausgewählt sein aus der Gruppe bestehend aus Fußulcera, wie insbesondere diabetische Fußulcera, Ulcus Cruris, Dekubitus, Tumorwunden, posttraumatische Wunden, Verbrennungswunden, Verätzungen, entzündliche Wunden, Abszesse, Amputationswunden und postoperative Wunden.

In weiterer Ausgestaltung der Erfindung handelt es sich bei den Wunden um Wunden von Patienten mit einer Herzkreislauf-Erkrankung, mit Diabetes mellitus Typ 1, mit Diabetes mellitus Typ 2 oder einer erektilen Dysfunktion. Insbesondere handelt es sich bei den Wunden um Wunden von Patienten mit einer blutdrucksenkenden, blutverdünnenden oder potenzsteigernden Medikation.

Weitere Vorteile und Merkmale der Erfindung ergeben sich aus den Ansprüchen sowie aus der nachfolgenden Beschreibung bevorzugter Ausführungsbeispiele der Erfindung. Es versteht sich, dass die Erfindung hierauf nicht beschränkt sein soll.

### BEISPIELTEIL

Es wurden folgende erfindungsgemäße Zusammensetzungen hergestellt:
1. Herstellung eines Hydrogels
- 0,1 Gew.-% Polyhexamethylenbiguanid (PHMB)
- 9 Gew.-% L-Arginin,
- 3,6 Gew.-% Hydroxyethylcellulose,
- 0,1 Gew.-% Alkylamidopropylbetain,
- 0,05 Gew.-% EDTA,
- 0,01 Gew.-% Tocopherolacetat,
- 15 Gew.-% Propylenglykol
- 5,0 Gew.-% Salzsäure und
- Rest Wasser.

Aussehen: transluzentes Gel
pH-Wert: 7,5
relative Dichte: 1,06 g/ml
Viskosität: 8619 mPas
2. Herstellung einer wässrigen Lösung
849,07 g Wasser wurden in einem geeigneten Mischreaktor vorgelegt. Ein Rührer wurde so eingestellt, dass sich auf der Wasseroberfläche eine leichte Bewegung zeigte. Anschließend wurden 90,00 g L-Arginin und 5,00 g Polyhexanid (20%ig) zugegeben. Darauf wurden 3,33 g Undecylenamidopropylbetain (30%ig) und 1,00 g Sorbit hinzugefügt. Zuletzt wurden 51,6 g Salzsäure (37%ig) zugegeben. Die Mischung wurde eine Stunde lang gerührt. Die erhaltene Lösung hatte die nachfolgende Zusammensetzung:

| | |
|---|---|
| L-Arginin | 9,0% (g/g) |
| Polyhexanid (20%ig) | 0,1% (g/g) |
| Undecylenamidoalkylbetain (30%ig) | 0,1% (g/g) |
| Sorbit | 0,1% (g/g) |
| Salzsäure (37%ig) | ad. pH 7 |
| ger. Wasser | ad. 100% |

3. Herstellung einer wässrigen Lösung
849,90 g Wasser wurden in einem geeigneten Mischreaktor vorgelegt. Ein Rührer wurde so eingestellt, dass sich auf der Wasseroberfläche eine leichte Bewegung zeigte. Anschließend wurden 90,00 g L-Arginin und 1,00 g Femotaxidine zugegeben. Darauf wurden 2,00 g Cocamidopropylbetain (50%ig) und 1,00 g Octandiol hinzugefügt. Zuletzt wurden 56,1 g Glykolsäure (70%ig) zugegeben. Die Mischung wurde eine Stunde lang gerührt. Die erhaltene Lösung hatte die nachfolgende Zusammensetzung:

| | |
|---|---|
| L-Arginin | 9,0% (g/g) |
| Femotaxidine | 0,1% (g/g) |
| Cocamidopropylbetain (50%ig) | 0,1% (g/g) |
| Octandiol | 0,1% (g/g) |
| Glykolsäure (70%ig) | ad. pH 7 |
| ger. Wasser | ad. 100% |

4. Herstellung einer Creme
Eine Wasserphase (A) und eine Fettphase (B) wurden jeweils auf 80°C erhitzt. Danach wurde die Fettphase (B) in die Wasserphase (A) gegeben und das erhaltene Gemisch wurde homogenisiert. Ab 70 °C wurde eine Phase (C) untergerührt. Die hierbei erhaltene Mischung wurde unter Rühren langsam abgekühlt.

### Phase A

| | |
|---|---|
| L-Arginin Hydrochlorid | 9,0% (g/g) |
| Polyhexanid (20%ig) | 0,1% (g/g) |
| Sorbit | 3,5% (g/g) |
| Zitronensäure | 0,5% (g/g) |
| ger. Wasser | ad. 100% |
| Phase B | |
| Crodamol CAP | 2,1% (g/g) |
| Stearinsäure | 0,6% (g/g) |
| Paraffinöl dünnflüssig | 8,0% (g/g) |
| TegoCare PS | 3,0% (g/g) |
| Glyceryl Stearat | 2,0% (g/g) |
| Phase C | |
| Sepigel 305 | 0,6% (g/g) |

## Patentansprüche

1. Zusammensetzung zur Anwendung bei der Behandlung von Wunden und/oder Vorbeugung oder Behandlung von Nekrosen, **dadurch gekennzeichnet, dass** die Zusammensetzung L-Arginin und/oder ein Salz von L-Arginin aufweist.

2. Zusammensetzung nach Anspruch 1, **dadurch gekennzeichnet, dass** das L-Arginin und/oder Salz von L-Arginin einen Anteil von 1 Gew.-% bis 40 Gew.-%, insbesondere 2 Gew.-% bis 20 Gew.-%, vorzugsweise 5 Gew.-% bis 15 Gew.-%; aufweist, bezogen auf das Gesamtgewicht der Zusammensetzung.

3. Zusammensetzung nach Anspruch 1 oder 2, **dadurch gekennzeichnet, dass** die Zusammensetzung ferner einen antimikrobiellen Wirkstoff, insbesondere ausgewählt aus der Gruppe bestehend aus Polyhexanid, Chlorhexidin, Octanid, Povidon-lod, Ethacridinlactat, Octenidin, Polylysin, Dihydrotriazinverbindungen wie Femotaxidin und Mischungen von wenigstens zwei der vorgenannten Wirkstoffe, aufweist.

4. Zusammensetzung nach einem der vorhergehenden Ansprüche, **dadurch gekennzeichnet, dass** die Zusammensetzung ferner ein zwitterionisches Tensid, vorzugsweise ein Betain-Tensid, insbesondere aus gewählt aus der Gruppe bestehend aus Caprylamidoalkylbetain, Caprinamidoalkylbetain, Undecylenamidoalkylbetain, Undecylamido-alkylbetain, Lauramidoalkylbetain, Lauryldimethylaminoessigsäurebetain, Stearinamidoalkyl-betain, Ricinolamidoalkylbetain, Cocamidoalkylbetain und Mischungen von wenigstens zwei der vorgenannten Tenside, aufweist.

5. Zusammensetzung nach einem der vorhergehenden Ansprüche, **dadurch gekennzeichnet, dass** die Zusammensetzung ferner ein Feuchthaltemittel, insbesondere ausgewählt aus der Gruppe bestehend aus Aloe Barbadensis-Extrakt, Glycerin, Polydextrose, Sorbit, Ethylenglykol, Polyethylenglykol, Propylenglykol, Butylenglykol, Pentylenglykol, Hexandiol, Octandiol, Glucose, Fructose, Glucuronsäure, Lactose, Milchsäure, Lactat, Lactulose, Saccharose, Hyaluronsäure, Xylitol, Xylose und Mischungen von wenigstens zwei der vorgenannten Feuchthaltemittel, aufweist.

6. Zusammensetzung nach einem der vorhergehenden Ansprüche, **dadurch gekennzeichnet, dass** die Zusammensetzung ferner ein Verdickungsmittel, insbesondere ausgewählt aus der Gruppe bestehend aus Celluloseether, Hydroxyethylcellulose, Carboxymethylcellulose, Hydroxypropylcellulose, Hydroxypropylmethylcellulose, Ethylhydroxyethylcellulose, Methylcellulose, Ethylcellulose, Methylethylcellulose, Natriumcarboxymethylcellulose, Chitosan, Xanthan, Stärke, Gelatine, Casein und Mischungen von wenigstens zwei der vorgenannten Verdickungsmittel, aufweist.

7. Zusammensetzung nach einem der vorhergehenden Ansprüche, **dadurch gekennzeichnet, dass** die Zusammensetzung eine wässrige Zusammensetzung ist, insbesondere einen Wasseranteil von 60 Gew.-% bis 99 Gew.-%, insbesondere 70 Gew.- % bis 95 Gew.-%, vorzugsweise 80 Gew.-% bis 90 Gew.-%, aufweist, bezogen auf das Gesamtgewicht der Zusammensetzung

8. Zusammensetzung nach einem der vorhergehenden Ansprüche, **dadurch gekennzeichnet, dass** die Zusammensetzung Folgendes aufweist:
- L-Arginin und/oder ein Salz von L-Arginin,
- einen antimikrobiellen Wirkstoff, vorzugsweise Polyhexanid,
- ein Betain-Tensid,
- ein Feuchthaltemittel und
- Wasser.

9. Zusammensetzung nach einem der vorhergehenden Ansprüche, **dadurch gekennzeichnet, dass** die Zusammensetzung, bezogen auf das Gesamtgewicht der Zusammensetzung, Folgendes aufweist:
- 5 Gew.-% bis 15 Gew.-% von L-Arginin und/oder eines Salzes von L-Arginin,
- 0,025 Gew.-% bis 0,25 Gew.-% eines antimikrobiellen Wirkstoffs, insbesondere Polyhexanid,
- 0,1 Gew.-% bis 1 Gew.-% eines Betain-Tensids,
- 0,1 Gew.-% bis 15 Gew.-% eines Feuchthaltemittels und
- Rest Wasser.

10. Zusammensetzung nach einem der vorhergehenden Ansprüche, **dadurch gekennzeichnet, dass** die Zusammensetzung Folgendes aufweist:
- L-Arginin und/oder ein Salz von L-Arginin,
- einen antimikrobiellen Wirkstoff, vorzugsweise Polyhexanid,
- ein Betain-Tensid,
- ein Feuchthaltemittel,
- ein Verdickungsmittel und
- Wasser.

11. Zusammensetzung nach einem der vorhergehenden Ansprüche, **dadurch gekennzeichnet, dass** die Zusammensetzung, bezogen auf das Gesamtgewicht der Zusammensetzung, Folgendes aufweist:
- 5 Gew.-% bis 15 Gew.-% von L-Arginin und/oder eines Salzes von L-Arginin,
- 0,025 Gew.-% bis 0,25 Gew.-% eines antimikrobiellen Wirkstoffs, insbesondere Polyhexanid,
- 0,1 Gew.-% bis 1 Gew.-% eines Betain-Tensids,
- 0,1 Gew.-% bis 15 Gew.-% eines Feuchthaltemittels,
- 1 Gew.-% bis 5 Gew.-% eines Verdickungsmittels und
- Rest Wasser.

12. Zusammensetzung nach einem der vorhergehenden Ansprüche, **dadurch gekennzeichnet, dass** die Zusammensetzung in Form eines Hydrogels vorliegt.

13. Zusammensetzung nach einem der vorhergehenden Ansprüche, **dadurch gekennzeichnet, dass** die Zusammensetzung für eine topische Verabreichung, insbesondere für eine Verabreichung auf die Wunden, hergerichtet ist.

14. Zusammensetzung nach einem der vorhergehenden Ansprüche, **dadurch gekennzeichnet, dass** es sich bei den Wunden um chronische Wunden, insbesondere ausgewählt aus der Gruppe bestehend aus Fußulcera, Ulcus Cruris, Dekubitus, Tumorwunden, posttraumatische Wunden, Verbrennungswunden, Verätzungen, entzündliche Wunden, Abszesse, Amputationswunden und postoperative Wunden, handelt.

15. Zusammensetzung nach einem der vorhergehenden Ansprüche, **dadurch gekennzeichnet, dass** es sich bei den Wunden um Wunden von Patienten mit einer Herzkreislauf-Erkrankung, mit Diabetes mellitus Typ 1, mit Diabetes mellitus Typ 2 oder einer erektilen Dysfunktion handelt.
